# EUROPÄISCHE PATENTSCHRIFT

(11) **EP 1 562 496 B1**
(45) Veröffentlichungstag und Bekanntmachung des Hinweises auf die Patenterteilung: **17.05.2006**
(21) Anmeldenummer: 03779995.4
(22) Anmeldetag: 19.11.2003
(51) Int. Cl.: A61B 17/34, A61B 17/02

(54) **BALLONTROKAR**
BALLOON TREPAN
TROCART A BALLON

(30) Priorität: 22.11.2002 DE 10254503
(43) Veröffentlichungstag der Anmeldung: 17.08.2005
(73) Patentinhaber: Karl Storz GmbH & Co. KG, 78532 Tuttlingen (DE)
(72) Erfinder: BACHER, Uwe, 78532 Tuttlingen (DE); SAUER, Michael, 78532 Tuttlingen (DE); OBERLÄNDER, Martin, 78532 Tuttlingen (DE)
(86) Internationale Anmeldenummer: PCT/EP2003/012959
(87) Internationale Veröffentlichungsnummer: WO 2004/047656

(56) Entgegenhaltungen:
- US-A- 5 868 662
- US-A- 5 925 058
- US-A- 6 142 945

## Beschreibung

Die Erfindung betrifft einen Ballontrokar bestehend aus einer Trokarhülse und einem Ballon, der ein distales Ende der Trokarhülse umgeben kann, wobei der Ballon zur Fixierung an der Trokarhülse durch ein Klemmelement gegen eine an der Trokarhülse umfänglich ausgebildete Stufe gepresst werden kann.

Ein derartiger Ballontrokar ist beispielsweise aus dem US-Patent US 5,607,443 bekannt.

Derartige Instrumente dienen zur Schaffung bzw. Aufrechterhaltung eines zu einer Operation notwendigen Raumes durch die Dilatation von Gewebe bzw. die Dissektion von Gewebeschichten.

Als Ballontrokar wird in der vorliegenden Schrift das Instrument inklusive Ballon bezeichnet, als Trokarhülse das Instrument ohne Ballon.

Bei der der Verwendung von Ballontrokaren zugrundeliegenden minimalinvasiven Operationstechnik wird der Ballontrokar durch einen Einschnitt ins Körperinnere eingeführt. Die Größe dieses Einschnitts wird so groß gewählt, dass das distale Ende der Trokarhülse umgeben von dem nicht aufgeblasenen Ballon eingeführt werden kann. Nach der Positionierung des Ballontrokars an der gewünschten Stelle im Körperinneren wird dann durch Aufblasen des Ballons mit einem Gas wie z. B. Luft oder Kohlenstoffdioxid, oder einer Flüssigkeit, wie z. B. Wasser, die Dilatation oder Dissektion des Gewebes durchgeführt.

Durch die Trokarhülse kann ein Endoskop eingeführt werden. Bei Verwendung eines Ballons, der zumindest teilweise aus durchsichtigem Material gefertigt ist, kann so der Operationsort beobachtet werden.

Ballontrokare kommen beispielsweise bei der Dilatation des extraperitonealen Raumes bei Hernienoperationen zum Einsatz.

Der verwendete Ballon kann aus dehnbaren Materialien gefertigt sein. Silicon hat sich beispielsweise als geeignet erwiesen. Geeignete Ballone sind derart geformt, dass sie über das distale Ende der Trokarhülse gestülpt und befestigt werden können. Beim Aufblasen vergrößert sich der Ballon durch Aufdehnen des elastischen Materials.

Auch Ballone aus inelastischen Materialien finden Verwendung. Bei diesen Ballontypen ist die Form des Ballons im aufgeblasenen Zustand bereits vorgegeben. Zum Einführen des Ballontrokars in das Körperinnere wird in diesem Fall der am distalen Ende der Trokarhülse befestigte Ballon zusammengefaltet um das distale Ende des Ballontrokars gelegt.

Vor der Operation wird der Ballon derart mit der Trokarhülse verbunden, dass ein Aufblasen nach dem Einführen in den Körper möglich ist. Die Verbindung zwischen Trokarhülse und Ballon muss also gegenüber dem beim Aufblasen auftretenden Druck stabil sein.

Zudem muss die Verbindung sicher gegen Verrutschen sein. D. h. die Position der Verbindung zwischen Trokarhülse und Ballon darf sich weder während des Einführens des Instruments in eine Körperhöhle noch während des Aufblasens des Ballons noch während der Entnahme des Instrumentes verschieben.

Ein Ballontrokar wird während Operationen häufig über die Befestigungsstelle des Ballons an der Trokarhülse hinaus in den Körper eingeführt. Deshalb ist eine weitere Anforderung, dass durch die Maßnahme zur Befestigung des Ballons an der Trokarhülse der Außendurchmesser des Ballontrokars nicht wesentlich vergrößert wird. Andernfalls müsste der zur Einführung des Ballontrokars vorgesehene Einschnitt in den Körper des Patienten unnötig vergrößert werden.

Wie im Folgenden erläutert wird, sind bereits Maßnahmen bekannt, um ein Verbindung zwischen Trokarhülse und Ballon zu erreichen.

In dem eingangs genannten US-Patent US 5,607,443 wird eine Maßnahme zur Fixierung eines Ballons an einer Trokarhülse offenbart. Das distale Ende der Trokarhülse weist eine äußere und eine innere Hülse auf. Der Ballon weist ein schlauchförmiges Ende auf, das durch Verklemmen zwischen den beiden Hülsen fixiert wird. Das schlauchförmige Ende wird gegen eine an der Innenseite der äußeren Hülse ausgebildete Stufe gepresst. Die Stufe ist am distalen Ende der äußeren Hülse angeordnet. D. h., dass sich durch die Stufe der Innendurchmesser der äußeren Hülse zu deren distalen Öffnung hin verkleinert. Das Ballonende wird zunächst umfänglich gegen diese Stufe gelegt. Im nächsten Schritt wird das Ballonende durch das Einführen der inneren Hülse in die äußere Hülse fixiert. An der Außenseite der inneren Hülse ist eine Stufe ausgebildet, die das Ballonende gegen die innere Stufe der äußeren Hülse presst.

Bei dieser Maßnahme erfordert die Montage, insbesondere das Einführen des Ballonendes in die äußere Hülse, einige Übung und ist zeitaufwendig. Zudem wird der Innendurchmesser der äußeren Hülse durch die eingeführte innere Hülse verengt. Die Stufe der inneren Hülse befindet sich innerhalb der äußeren Hülse. Somit wird bei vorgegebenem Außendurchmesser der Raum zum Einführen von z. B. Endoskopen verringert.

In dem genannten US-Patent US 5,607,443 wird eine weitere Maßnahme der Fixierung des Ballons an der Trokarhülse durch eine Schlauchschelle beschrieben. Eine häufig zur Bestückung von Ballontrokaren verwendete Form von Ballonen weist zur Öffnung des Ballons hin ein schlauchförmiges Ende auf. In dieses schlauchförmige Ende wird die Trokarhülse eingeführt. Durch eine das schlauchförmige Ende umgebende Schlauchschelle wird der Ballon dann zur Fixierung gegen die Oberfläche der Trokarhülse gepresst.

Bei dieser Art der Fixierung des Ballons ist es nachteilig, dass die Schlauchschelle den Außendurchmesser der Trokarhülse vergrößert. Ein Einführen des Ballontrokars in den Körper über die Befestigungsstelle des Ballons an der Trokarhülse hinaus würde demnach einen größeren Einschnitt in den Körper des Patienten erfordern.

In dem Deutschen Gebrauchsmuster DE 298 06 831 U1 wird eine weitere Maßnahme zur Befestigung des schlauchförmigen Endes des Ballons an der Trokarhülse beschrieben. Um das an der Trokarhülse anliegende Ende des Ballons wird eine Bandage aus nicht dehnbarem Material gelegt, deren Enden in einem Kontaktbereich vorzugsweise durch Verkleben miteinander verbunden werden.

An dieser Maßnahme ist nachteilig, dass eine fehlerhafte Montage der Bandage leicht möglich ist. Der Anwender muss sowohl auf einen festen Sitz der Bandage als auch auf eine korrekte Verbindung der Enden der Bandage im Kontaktbereich achten. Ein mehrfaches Öffnen und Schließen einer derartigen Bandage, um z. B. den Sitz des Ballons zu korrigieren, ist in der Regel nicht möglich, da es sich um ein Einwegprodukt handelt. Die Spannung der Bandage, d. h. die Kraft, mit der das Ballonende gegen die Trokarhülse gepresst wird, kann bei der Montage, wenn dies überhaupt möglich ist, nur sehr ungenau eingestellt werden.

Eine weitere bekannte Maßnahme ist das Fixieren des Ballons an der Trokarhülse durch einen Gummiring. Auch bei dieser Maßnahme weist der Ballon ein schlauchförmiges Ende auf, in welches die Trokarhülse eingeführt wird. Der Gummiring wird über die Trokarhülse und das Ende des Ballons gespannt. Diese Methode ist insbesondere dann problematisch, wenn Ballone aus elastischen Materialien verwendet werden. Besteht das schlauchförmige Endes eines derart befestigten Ballons aus elastischem Material, so kann, bei Belastung des Ballons durch Innendruck, der Gummiring aufgedehnt werden.

Besteht der Ballon bzw. zumindest die Öffnung des Ballons aus elastischem Material, so ergibt sich eine weitere, bereits bekannte Möglichkeit zur Fixierung eines Ballons an einer Trokarhülse. Der Innendurchmesser der Öffnung des Ballons wird kleiner ausgebildet, als der Außendurchmesser des Abschnitts der Trokarhülse, an dem der Ballon befestigt wird. Durch die Elastizität des Materials des Ballons kann der Durchmesser durch Aufdehnen derart erweitert werden, dass ein Aufschieben des Ballons auf die Trokarhülse möglich wird. Die Unterschiede zwischen dem Innendurchmesser der Öffnung des Ballons und des Außendurchmessers der Trokarhülse werden derart gewählt, dass die Öffnung des Ballons mit der gewünschten Stabilität an der Trokarhülse anliegt.

Die Montage des Ballons ist bei dieser Maßnahme mühsam, der Ballon kann beim Aufschieben auf die Trokarhülse leicht beschädigt werden. Diese Maßnahme genügt ohne zusätzliche Vorkehrungen, wie beispielsweise eine der oben genannten, meist nicht der geforderten Stabilität der Verbindung.

US 5,925,058 offenbart einen Ballontrokar gemäß dem Oberbegriff des Anspruch 1.

Aufgabe der Erfindung ist daher, einen Ballontrokar zu schaffen, der unter Vermeidung der genannten Nachteile eine schnelle und sichere Befestigung des Ballons an der Trokarhülse ermöglicht.

Erfindungsgemäß wird diese Aufgabe dadurch gelöst, dass das Klemmelement durch Drehung zumindest eines Teils des Klemmelements um eine Längsachse der Trokarhülse in Längsrichtung zur Trokarhülse bewegbar ist.

Die Umsetzung der Drehbewegung in Längsrichtung kann beispielsweise durch umfänglich an der Trokarhülse angeordnete Steuerkurven bzw. Gewinde erfolgen. Durch Drehung zumindest eines Teils des Klemmelements wird dieses entlang der Steuerkurve bzw. des Gewindes bewegt.

Diese Maßnahme hat den Vorteil, dass das Klemmelement auf einfache Weise in jede beispielsweise durch die Steuerkurve bzw. durch das Gewinde vorgegebene Position längs zur Trokarhülse gebracht werden kann.

Es ist beispielsweise auf einfache Weise möglich, ein kontinuierliches Verschieben des Klemmelementes in Längsrichtung der Trokarhülse zu ermöglichen. Dies erlaubt es, die Kraft, mit welcher der Ballon durch das Klemmelement gegen die Stufe gepresst wird, beliebig einzustellen. So können auch Ballone, deren Ballonenden in unterschiedlicher Stärke ausgebildet sind, problemlos mit dem gewünschten Anpressdruck fixiert werden.

In einer weiteren Ausgestaltung der Erfindung ist das Klemmelement auf jener Seite der Stufe an der Trokarhülse fixierbar, von welcher der Ballon gegen die Stufe gepresst werden kann.

Bei dieser Maßnahme wird der über das distale Ende der Trokarhülse gestülpte und an der Stufe ausgerichtete Ballon durch das Klemmelement wie folgt fixiert. Das Klemmelement wird in eine Position bewegt, in welcher der Ballon gegen die Stufe der Trokarhülse gepresst wird. Der Ballon kommt beim Anpressen nur mit einem Abschnitt des Klemmelements in Kontakt, während ein weiterer Abschnitt des Klemmelementes zur Fixierung an der Trokarhülse dient. Das Klemmelement ist in dieser Position derart an der Trokarhülse fixiert, dass eine ausreichende Kraft zum Anpressen des Ballons gewährt ist. In speziellen Ausgestaltungen kann beispielsweise vorgesehen werden, dass der Anpressdruck des Klemmelementes individuell einstellbar ist.

Die Stufe, gegen welche der Ballon gepresst wird, kann in die proximale oder in die distale Richtung der Trokarhülse zeigen.

Das Klemmelement wird demnach proximalseitig oder distalseitig der Stufe fixiert.

Diese Maßnahme hat den Vorteil, dass eine fehlerfreie Fixierung des Ballons an der Trokarhülse vereinfacht wird. Da die an der Trokarhülse ausgebildete Stufe nicht notwendigerweise vom Klemmelement umgeben werden muss, kann als weiterer Vorteil eine Vergrößerung des Innendurchmessers der Trokarhülse bei gleichem Außendurchmesser erreicht werden.

Das mit der Trokarhülse verbundene Klemmelement wird während der Bewegung entlang der Trokarhülse zum Fixieren des Ballons sicher geführt, so dass eine Fehlbedienung nur schwer möglich ist. Die Fixierung kann beispielsweise auch, je nach Ausgestaltung, einfach und reversibel gelöst werden, um den Sitz des Ballons zu korrigieren.

In einer weiteren Ausgestaltung der Erfindung ist das Klemmelement zumindest abschnittsweise ring- oder röhrenförmig ausgebildet, wobei dessen Querschnitt zumindest abschnittsweise eine Öffnung aufweist, deren Form dem äußeren Querschnitt der Trokarhülse entspricht.

Diese Maßnahme hat den Vorteil, dass das Klemmelement auf einfache Weise mit der Trokarhülse verbunden werden und gleichzeitig als in axialer Richtung bewegbar ausgestaltet werden kann. Ein weiterer Vorteil diese Ausgestaltung ist, dass der zu klemmende Abschnitt des Ballonendes nicht in das Klemmelement eingebracht und umfänglich gegen dessen Innenseite gelegt werden muss.

Bei dieser Maßnahme kann das Klemmelement beispielsweise derart ausgestaltet sein, dass es das distale Ende der Trokarhülse in jenem Bereich umschließt, der bei einer Operation in den Körper eingeführt wird und der nicht vom Ballon umschlossen wird. Dies hat den weiteren Vorteil, dass dieser Bereich der Trokarhülse während der Operation vor Verschmutzung geschützt wird. Beim Einführen des distalen Endes des Ballontrokars während einer Operation werden durch diese Maßnahme nur der Ballon und das Klemmelement verschmutzt. Dies verringert den Reinigungsaufwand erheblich. Statt des kompletten Ballontrokars muss so nur das Klemmelement gereinigt werden, da der Ballon in den meisten Fällen zur einmaligen Benutzung ausgelegt ist. Zudem eröffnet sich die Möglichkeit, den relevanten Teil des Klemmelementes oder das komplette Klemmelement als Einwegartikel auszubilden.

In Verbindung mit dieser Maßnahme kann es beispielsweise von Vorteil sein, dass die Stufe durch eine Erweiterung des Außendurchmessers der Trokarhülse ausgebildet wird. Es kann weiterhin vorteilhaft sein, wenn der Außendurchmesser des röhrenförmigen Klemmelementes mit dem Außendurchmesser des erweiterten Abschnitts der Trokarhülse übereinstimmt. Wird das derart gestaltete Klemmelement gegen die Stufe geschoben, entsteht umfänglich ein Spalt, in welchen das Ballonende eingeklemmt werden kann. Vorteilhaft ist, dass der Außendurchmesser der Trokarhülse vom distalen Ende bis über die Befestigungsstelle des Ballons hinaus konstant ist. Das Einführen des Ballontrokars durch einen Einschnitt in eine Körperhöhle wird so durch keine Vorsprünge behindert.

In einer weiteren Ausgestaltung der Erfindung weist das Klemmelement umfänglich eine Kontaktfläche auf, die zur Fixierung des Ballons an dessen Oberfläche anliegt.

Die Kontaktfläche kann sowohl an der Oberfläche der Innenseite als auch an der Oberfläche der Außenseite des Ballons anliegen.

Diese Maßnahme hat den Vorteil, dass die Stabilität der Verbindung zwischen Ballon und Trokarhülse erhöht wird und sie sicherer der Belastung beim Aufblasen des Ballons standhält. Das Risiko, dass beispielsweise während der Operation Gas oder Flüssigkeit, d. h. das Medium, welches zum Aufblasen des Ballons Verwendung findet, aus dem Innenraum des Ballons in die Körperhöhle entweicht, wird minimiert.

In einer weiteren Ausgestaltung der Erfindung ist die Kontaktfläche als Stirnfläche des Klemmelements ausgebildet.

Diese Maßnahme hat den Vorteil, dass sie mit geringem Aufwand realisierbar ist. Das Klemmelement kann einstückig ausgebildet, aber auch aus mehreren Teilen zusammengesetzt sein. In Richtung zur Kontaktfläche kann die Wandstärke des Klemmelements zunehmen, um somit die Ausgestaltung einer größeren Kontaktfläche zu ermöglichen.

Das Klemmelement kann beispielsweise auch einen Ring aufweisen, wobei die Kontaktfläche die Stirnfläche des Ringes ist. Die Kontaktfläche kann dann durch die Form des Ringes gestaltet werden.

In einer weiteren Ausgestaltung der Erfindung ist die Kontaktfläche des Klemmelementes zumindest abschnittsweise gegen die Oberfläche der Trokarhülse geneigt.

Diese Maßnahme hat den Vorteil, dass der Ballon durch das Klemmelement effektiver an der Trokarhülse fixiert werden kann. Diese Maßnahme ist beispielsweise in Verbindung mit der weiter unten beschriebenen Ausgestaltung der Erfindung von Vorteil, in der der Rand der Öffnung des Ballons als Wulst ausgestaltet ist. Das Klemmelement und eine senkrechte oder ebenfalls gegen die Oberfläche der Trokarhülse geneigte Stufe bilden einen ringförmigen Hohlraum. In diesen Hohlraum kann der Wulst des Ballons bei entsprechender Größe sicher eingeklemmt werden.

In einer weiteren Ausgestaltung der Erfindung ist die Stufe zumindest abschnittsweise gegen die Oberfläche der Trokarhülse geneigt.

Der Vorteil dieser Maßnahme wurde bereits bei der oben genannte Ausgestaltung, dass die Kontaktfläche des Klemmelementes zumindest abschnittsweise gegen die Oberfläche der Trokarhülse geneigt ist, beschrieben. Selbstredend ist auch eine Kombination beider Ausgestaltungen möglich.

In einer weiteren Ausgestaltung der Erfindung ist die Stufe drehbar zur Längsachse der Trokarhülse gelagert.

Diese Maßnahme ist insbesondere in Verbindung mit der Ausgestaltung, bei der das Klemmelement durch Drehung zumindest eines Teils des Klemmelements um die Längsachse der Trokarhülse in Längsrichtung zur Trokarhülse bewegt wird, von Vorteil. Wird bei dieser Ausgestaltung vorgesehen, dass das komplette Klemmelement, zumindest aber dessen Kontaktfläche gedreht wird, so verhindert die drehbare Lagerung der Stufe beim Festklemmen des Ballons eine Scherung des Abschnitts des Ballonendes, der sich zwischen Kontaktfläche und Stufe befindet.

Die drehbar gelagerte Stufe kann auch in Längsrichtung zur Trokarhülse fixierbar sein, um ein Verlieren derselben zu vermeiden.

In einer weiteren Ausgestaltung der Erfindung weist das Klemmelement eine Handhabe zu dessen Betätigung auf.

Diese Maßnahme hat den Vorteil, dass das Klemmelement ergonomisch günstig an leicht erkennbarer Stelle zu bedienen ist.

In einer weiteren Ausgestaltung der Erfindung ist die Handhabe als Drehring ausgebildet, durch dessen Drehung das Klemmelement in axialer Richtung bewegt wird.

Diese Maßnahme hat den Vorteil, dass die Position des Klemmelements ergonomisch günstig und auf einfache Weise einstellbar ist.

In einer weiteren Ausgestaltung der Erfindung ist die Handhabe am proximalen Ende der Trokarhülse angeordnet.

Diese Maßnahme hat den Vorteil, dass die Handhabe bei einer Operation nicht in den Körper eingeführt werden muss. Sie kann somit ergonomisch vorteilhaft mit größerem Außendurchmesser ausgestaltet werden kann, ohne dass dies für die Funktion des Ballontrokares nachteilig wäre. Wenn die Handhabe im distalen Bereich angeordnet ist, der bei einer Operation eventuell in den Körper eingeführt wird, so muss die Handhabe möglichst schlank und glatt ausgebildet sein.

In einer weiteren Ausgestaltung der Erfindung ist das Klemmelement zumindest abschnittsweise innerhalb des Ballons angeordnet.

Diese Maßnahme hat den Vorteil, dass Klemmelement und Ballon auf einfache Weise miteinander Verbunden werden können, bevor das Klemmelement mit der Trokarhülse verbunden wurde. Klemmelement und Ballon können somit beispielsweise als vormontierte Einheit zur Verfügung gestellt werden. Diese Einheit kann als Einwegartikel vorgesehen sein.

In einer weiteren Ausgestaltung der Erfindung wird das Klemmelement zur Fixierung des Ballons in Richtung des proximalen Endes der Trokarhülse bewegt.

Diese Bewegung zum Schließen des Klemmelementes kommt insbesondere dann zum Einsatz, wenn, wie in einer oben beschriebenen Ausgestaltung, das Klemmelement zumindest abschnittsweise innerhalb des Ballons angeordnet ist.

Diese Maßnahme hat den Vorteil, dass die Montage von Ballon und Klemmelement weiter vereinfacht wird.

In einer weiteren Ausgestaltung der Erfindung ist das Klemmelement als eine distale Führung der Trokarhülse ausgebildet.

Diese Maßnahme hat den Vorteil, dass der Aufbau des Ballontrokars vereinfacht werden kann. Durch die geringere Anzahl an Bauteilen wird schnelleres und kostengünstigeres Zerlegen und Reinigen der Trokarhülse ermöglicht.

Diese Maßnahme ist auch dann von Vorteil, wenn das als distale Führung ausgebildete Klemmelement auf einfache Weise, beispielsweise durch Verschrauben, mit der Trokarhülse verbunden bzw. von dieser abgenommen werden kann. Dies ermöglicht es, den Ballon bereits vor der Montage der distalen Führung an der Trokarhülse mit der distalen Führung zu verbinden. Dies erleichtert die Montage des Ballons erheblich. Die distale Führung und der Ballon können als vormontierte Einheit zur Verfügung gestellt werden und ggf. als Einwegartikel ausgebildet sein. Dies vereinfacht die Reinigung und Sterilisation des Ballontrokars, da die vormontierte Einheit bereits steril bereitgestellt werden kann.

Das als distale Führung ausgebildete Klemmelement kann auch aus transparentem Material, wie beispielsweise transparentem Kunststoff, ausgebildet sein. Durch diese Maßnahme wird auf einfache Weise auch im Bereich der distalen Führung die Beobachtung des zu untersuchenden Hohlraumes durch ein Endoskop möglich.

In einer weiteren Ausgestaltung der Erfindung wird eine Öffnung des Ballons durch eine Montagehilfe aufgedehnt, wobei der Innendurchmesser der Montagehilfe größer oder gleich dem Außendurchmesser der Trokarhülse ist.

Durch diese Maßnahme wird die Montage eines Ballons, dessen Öffnung zum Einführen einer Trokarhülse aufzudehnen ist, vereinfacht. In die Öffnung des Ballons wird eine Montagehilfe eingebracht. Diese ist beispielsweise als Rohr ausgebildet, dessen Innendurchmesser etwas größer ist als der Außendurchmesser des distalen Endes der Trokarhülse. Die Öffnung des Ballons kann beispielsweise als schlauchförmiges Ende ausgebildet sein. In diesem Fall, wird die Länge des Rohres kürzer gewählt als die Länge des schlauchförmigen Endes des Ballons. Der überstehende Abschnitt des schlauchförmigen Endes wird vor der Montage des Ballons auf das Rohr aufgerollt. Nach dem Einführen des distalen Endes der Trokarhülse in das mit dem Ballon bestückte Rohr bis zur Stelle, an welcher die Klemmung erfolgen soll, wird der Ballon abgerollt. Danach wird dessen Ende zwischen Stufe und Klemmelement derart angeordnet, dass eine Fixierung durch das Klemmelement erfolgen kann. Das Rohr verbleibt während der Benutzung des Ballontrokars im Ballon. Um eine Behinderung der Sicht mit einem in die Trokarhülse eingeführten Endoskop zu vermeiden, kann das Rohr aus einem transparenten Material, beispielsweise einem transparenten Kunststoff, gefertigt sein.

Es ist ein Vorteil dieser Maßnahme, dass sie auf einfache Weise ermöglicht, einen Ballon durch Aufdehnen der Ballonöffnung an der Trokarhülse zu fixieren. Der so fixierte Ballon kann durch weitere der bekannten Methoden aber auch durch die erfindungsgemäße Methode zusätzlich fixiert werden. Folgende Möglichkeit bietet sich beispielsweise, wenn das Klemmelement proximalseitig der Stufe fixiert wird. Wird die Öffnung des Ballons oder des schlauchförmigen Endes des Ballons, durch welche die Trokarhülse eingeführt wird, kleiner gewählt als der Außendurchmesser des distalen Endes der Trokarhülse, so muss die Öffnung des Ballons bei der Montage, d. h. beim Einführen der Trokarhülse, aufgedehnt werden. Nach dem Überwinden der Stufe wird die Dehnung der Öffnung wieder reduziert. Somit ist der Ballon schon vor dem Schließen des Klemmelementes an der Trokarhülse vorläufig fixiert. Ist das Klemmelement distalseitig der Stufe fixiert, kann statt der Stufe beispielsweise bei der Montage des Ballons auch ein entsprechend ausgestaltetes Klemmelement durch Aufdehnen der Ballonöffnung überwunden werden, um eine vorläufige Fixierung des Ballons zu bewirken.

In einer weiteren Ausgestaltung der Erfindung ist ein Rand eine Öffnung des Ballons als Wulst ausgebildet.

Diese Maßnahme hat den Vorteil, dass der Wulst eine stabilere Fixierung des Ballonendes an der Trokarhülse ermöglichen kann. Dies ist insbesondere dann der Fall, wenn wie in oben beschriebenen Ausgestaltungen die Stufe der Trokarhülse und/oder die Kontaktfläche des Klemmelementes gegen die Trokarhülse geneigt ist/sind. Beim Schließen des Klemmelementes entsteht so eine sich nach außen verjüngende Spalte. Es kann für die Befestigung des Ballons vorteilhaft sein, wenn die ringförmige Verdickung innerhalb dieser Spalte Platz findet. Zudem ist das Ballonende durch die Verdickung gegen Einreißen geschützt.

Es versteht sich, dass die bisher genannten und die im Folgenden noch zu erläuternden Merkmale der Erfindung nicht nur in den beschriebenen, sondern auch in weiteren Kombinationen oder einzeln Verwendung finden können, ohne den Rahmen der vorliegenden Erfindung zu verlassen.

Die Erfindung wird im Folgenden anhand einiger ausgewählter Ausführungsbeispiele und den Zeichnungen näher beschrieben und erläutert.

Es zeigen:
Fig. 1: Seitenansicht, teilweise im Längsschnitt aufgebrochen, eines erfindungsgemäßen Ballontrokars gemäß einem ersten Ausführungsbeispiel;
Fig. 1A: Darstellung gemäß Fig. 1 mit geöffnetem Klemmelement;
Fig. 1B: Vergrößerung des mit IB bezeichneten Ausschnitts der Fig. 1A;
Fig. 1C: Vergrößerung des mit IC bezeichneten Ausschnitts der Fig. 1A;
Fig. 2: Vergrößerung des mit A bezeichneten Ausschnitts der Fig. 1 in perspektivischer Darstellung;
Fig. 3: Vergrößerung des mit B bezeichneten Ausschnitts der Fig. 1 in perspektivischer Darstellung, statt des vollständigen Ballons ist nur dessen Wulst mit angedeutetem schlauchförmigem Abschnitt gezeigt;
Fig. 4: perspektivische Darstellung eines erfindungsgemäßen Bauteils des Klemmelements gemäß dem ersten Ausführungsbeispiel;
Fig. 5: Längsschnitt eines erfindungsgemäßen Ballons mit Montagehilfe gemäß einem zweiten Ausführungsbeispiel;
Fig. 6: perspektivische Darstellung eines erfindungsgemäßen Bauteils des Klemmelements gemäß einem dritten Ausführungsbeispiel;
Fig. 7: Längsschnitt eines erfindungsgemäßen Ballons mit distaler Führung gemäß dem dritten Ausführungsbeispiel;
Fig. 8: Abschnitt einer Seitenansicht, teilweise im Längsschnitt aufgebrochen, einer erfindungsgemäßen Trokarhülse gemäß dem dritten Ausführungsbeispiel.

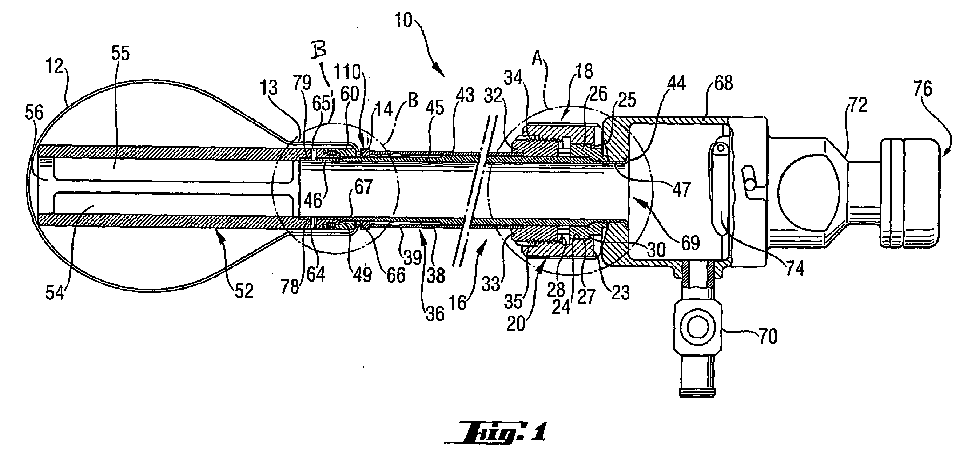

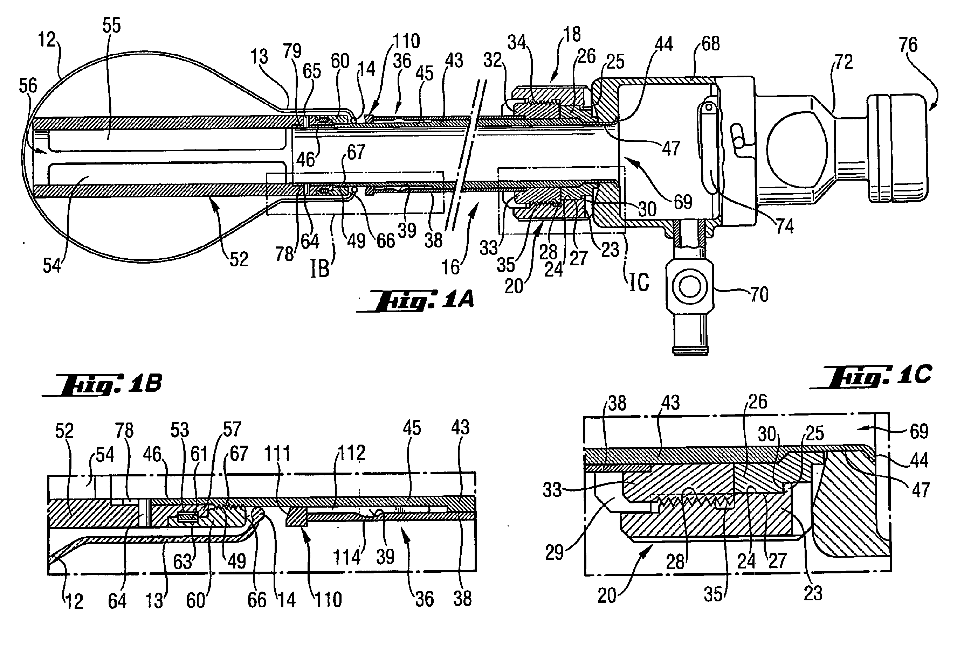

Einen Ballontrokar 10 eines ersten Ausführungsbeispiels bestehend aus einem Ballon 12 und einer Trokarhülse 16 zeigen Fig. 1 und Fig. 1A im Längsschnitt. Der Ballon 12 weist an der Öffnung einen als ringförmige Verdickung ausgebildeten Wulst 14 auf, der durch das Klemmelement 18 am Ballontrokar 10 fixiert wird.

Der Ballon 12 ist aus elastischem auf Silicon basierendem Material gefertigt, welches im gedehnten Zustand transparent ist. Der Ballon 12 hat, ausgehend von dem Wulst 14, einen schlauchförmigen Abschnitt 13, gefolgt von einem nicht näher bezeichneten tropfenförmigen Ballonkörper.

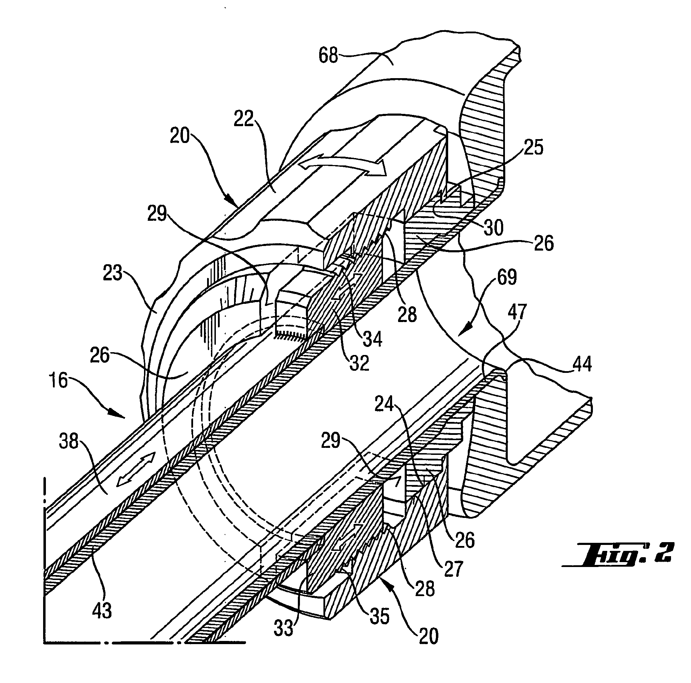

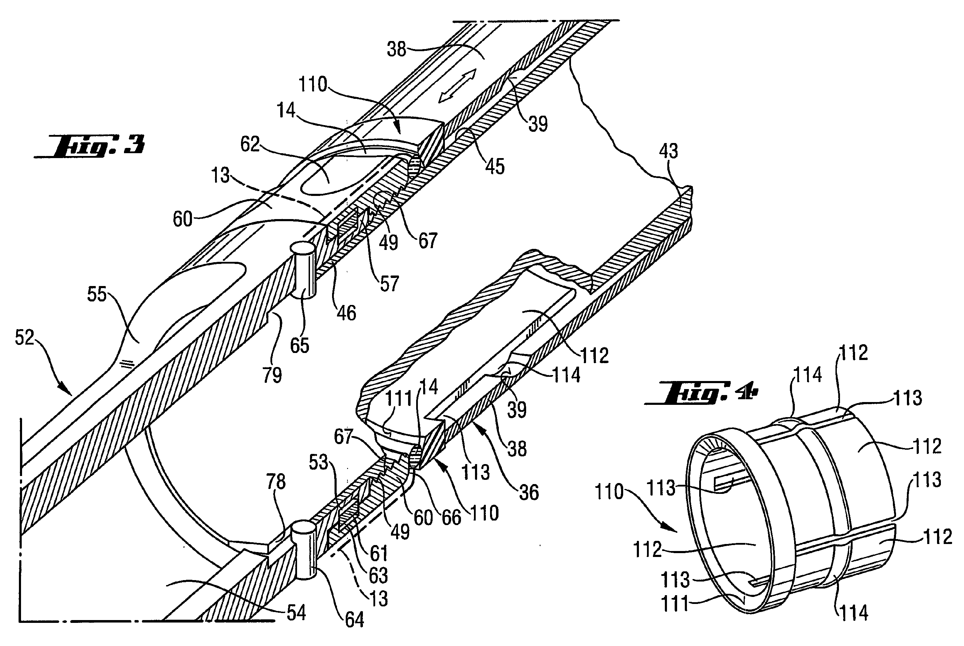

Fig. 2 und Fig. 3 zeigen vergrößerte perspektivische Ansichten der mit A und B bezeichneten Ausschnitte der Fig. 1.

Handhabe 20 und Hülse 36 sind Teile des Klemmelementes 18. Das Klemmelement 18, wie in Fig. 1 und ausschnittsweise in Fig. 2 gezeigt, wird durch Betätigung einer Handhabe 20 in axialer Richtung bewegt. Die Handhabe 20 weist einen Drehring 23 auf, der auf einem Lager 26 drehbar gelagert ist, welches die Haupthülse 43 der Trokarhülse 16 umgibt und mit dieser durch Verschweißen verbunden ist.

Ein Verschieben des Drehrings 23 in Längsrichtung zum proximalen Ende hin wird durch den Ventilkörper 68 verhindert. Ein Verschieben in distale Richtung wird durch Anschlag 25 des Drehrings 23 und die Stufe 30 des Lagers 26 begrenzt.

Haupthülse 43 und Ventilkörper 68 sind durch Formschluss miteinander verbunden. Der Durchmesser der Öffnung 69 des Ventilkörpers 68 entspricht im distalen Bereich der Öffnung 69 dem Außendurchmesser der Haupthülse 43. Im proximalen Bereich ist der Durchmesser der Öffnung 69 erweitert. Der Ventilkörper 68 wird in distaler Richtung durch das Lager 26 und in proximaler Richtung durch den gemäß dem erweiterten Innendurchmesser der Öffnung 69 nach außen gebördelten Rand 44 der Haupthülse 43 fixiert. Die Haupthülse 43 weist am proximalen Ende eine Verjüngung 47 des Außendurchmessers auf. Da der Innendurchmesser der Haupthülse 43 konstant bleibt, wird so eine Verminderung der Wandstärke erreicht, die das Bördeln des Randes 44 ermöglicht.

Das Lager 26 weist umfänglich eine Gleitfläche 27 auf, die proximalseitig durch eine Stufe 30 begrenzt wird. Der Außendurchmesser des Lagers 26 im Bereich der Gleitfläche 27 entspricht dem Innendurchmesser im Bereich der Gleitfläche 24 des Drehrings 23.

Im Drehring 23 ist ein Innengewinde 28 ausgebildet, durch welches beim Drehen des Drehringes 23 die Mitnahmeelemente 32 und 33 längs der Trokarhülse 16 bewegt werden. Die Form der Mitnahmeelemente 32 und 33 kann als Segment eines die Trokarhülse umgebenden Ringes beschrieben werden. Diese Mitnahmeelemente 32 und 33 sind gegenüberliegend um die Trokarhülse 16 zwischen der Oberfläche des Außenrohres 38 und der Innenseite des Drehrings 23 angeordnet. Auf der Außenseite weisen die Mitnahmeelemente Gewindesegmente 34 und 35 auf, die in das Innengewinde 28 des Drehrings 23 greifen. Die Mitnahmeelemente 32 und 33 sind mit dem Außenrohr 38 der Hülse 36 verbunden.

Das Außenrohr 38 umgibt den Bereich der Trokarhülse zwischen der proximalseitig angeordneten Handhabe 20 und dem Ballon 12. Außenrohr 38 und Ring 110 bilden die Hülse 36.

Die Mitnahmeelemente 32 und 33 bewegen sich in Richtung der Längsachse der Trokarhülse 16 in Kerben des Lagers 26. Eine der beiden Kerben ist in Fig. 2 dargestellt und mit der Bezugsziffer 29 bezeichnet. Durch Drehung des Drehrings 23 erfolgt somit eine Bewegung der Hülse 36 in Längsrichtung, ohne dass die Hülse 36 selbst eine Drehung erfährt.

Zur leichteren Handhabung sind, wie in Fig. 2 dargestellt, auf der Außenseite des Drehringes 23 mehrere Mulden eingearbeitet, die ein Abrutschen bei dessen Betätigung vermeiden. Exemplarisch ist eine Mulde mit der Bezugsziffer 22 (Fig. 2) bezeichnet.

Die Kontaktfläche 111 (Fig. 1B und Fig. 3) des Klemmelements 18, die zur Fixierung des Ballons 12 an dessen Oberfläche anliegt, ist Teil des Ringes 110.

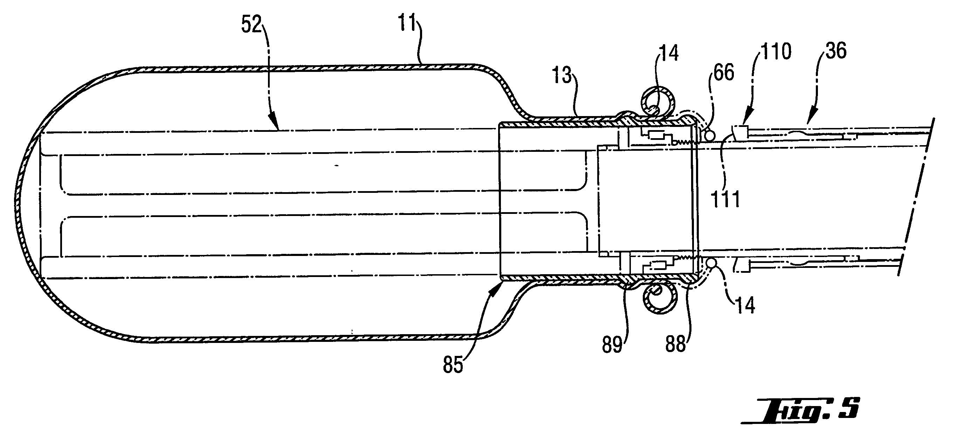

Eine perspektivische Darstellung des Ringes 110 zeigt Fig. 4. Die Stirnfläche des Ringes 110 dient als Kontaktfläche 111, welche zur Fixierung des Ballons gegen dessen Oberfläche gepresst werden kann. Die Kontaktfläche 111 ist gegen die Oberfläche der Trokarhülse 16 geneigt.

Die vier Laschen des Rings 110, die in ihrer Gesamtheit mit der Bezugsziffer 112 bezeichnet werden (Fig. 4), greifen zwischen die Außenseite der Haupthülse 43 und die Innenseite des Außenrohres 38. Die Laschen 112 werden durch die Kerben, die in ihrer Gesamtheit mit der Bezugsziffer 113 bezeichnet werden, voneinander getrennt. Der zum Einbringen der Laschen 112 benötigte Zwischenraum wird durch die Verjüngung 45 des Außendurchmessers der Haupthülse 43 geschaffen (Fig. 3).

Die umfänglich an den Außenseiten der Laschen ausgebildeten Erhebungen, die in ihrer Gesamtheit mit der Bezugsziffer 114 bezeichnet werden, greifen zur axialen Fixierung des Ringes 110 in die Ringnut 39, die an der Innenseite des Außenrohres 38 ausgebildet ist, ein. Die Laschen 112 sind elastisch, so dass sie bei der Montage des Ringes 110 bis zum Einrasten der Erhebungen 114 in die Ringnut 39 nach innen gebogen werden können. Ring 110 ist aus Kunststoff, in einem weiteren Ausführungsbeispiel aus Stahl, hergestellt.

Die Stufe 66, gegen die der Ballon 12 durch das Klemmelement 18 gepresst wird, ist, wie in Fig. 1 dargestellt, Teil des Überwurfrings 60. Dieser dient gleichzeitig zur Befestigung des Käfigs 52 an der Haupthülse 43. Überwurfring 60 ist drehbar mit Käfig 52 verbunden. Diese Verbindung erfolgt durch einen Sprengring 61 (Fig. 3), der sowohl in der auf der Außenseite des Käfigs 52 umlaufenden Nut 53 als auch in der in die Innenseite des Überwurfringes 60 eingearbeiteten Nut 63 angeordnet ist.

Zur Befestigung des Käfigs 52 durch den Überwurfring 60 an der Haupthülse 43 wird, wie in Fig. 3 dargestellt, der Überwurfring 60 durch das Innengewinde 67 und das Außengewinde 49 an der Haupthülse 43 festgeschraubt. Auf der Außenseite des Überwurfringes 60 sind Mulden 62 ausgebildet, um dessen Handhabung zu erleichtern.

Im Bereich des distalen Endes 46 der Haupthülse 43, welches in den Käfig 52 ragt, ist der Außendurchmesser gegenüber der Verjüngung 45 weiter reduziert. Der Innendurchmesser des Käfigs 52 ist an dessen proximalem Ende 57 erweitert, so dass er das distale Ende 46 der Haupthülse 43 passgenau aufnimmt. Der Innendurchmesser der gesamten Trokarhülse 16 ist konstant.

Käfig 52 wird, wie in Fig. 1 und 3 dargestellt, durch die Positionsstifte 64 und 65 gegen Verdrehen gesichert. Diese sind derart an dem Käfig 52 angebracht, dass sie in dessen Innenraum ragen. Wenn der Käfig 52 an der Haupthülse 43 befestigt ist, ragen die Positionsstifte 64 und 65 in die Kerben 78 und 79 der Haupthülse 43.

Käfig 52 hat umfänglich vier Öffnungen, von denen zwei in den Zeichnungen dargestellt und mit den Bezugsziffern 54 und 55 bezeichnet sind. Diese Öffnungen und die Öffnung 56 am distalen Ende des Käfigs 52 dienen zur Beobachtung mit einem, in den Figuren nicht dargestellten, in die Trokarhülse 16 eingeführten Endoskop.

Der Ventilkörper 68 ist, wie in Fig. 1 und Fig. 1A dargestellt, mit einer Ventilklappe 74 versehen. Mit dem Ventilkörper 68 ist ein Einlass 72 verbunden. Die proximale Öffnung 76 des Einlasses 72 dient zum Einführen eines nicht dargestellten Endoskops. Ein am Ventilkörper 68 angebrachter Spül-Saugstutzen 70 dient zum Aufblasen des Ballons mit einer Flüssigkeit oder mit einem Gas oder zum Entleeren.

Zur Montage des Ballons wird der schlauchförmige Abschnitt 13 des Ballons 12 über den Käfig 52 und den Überwurfring 60 gestülpt. Bei geöffnetem Klemmelement 18 wird der Wulst 14 des Ballons 12 proximalseitig zur Stufe 66 angeordnet, so dass er die Verjüngung 45 der Haupthülse 43 umgibt und gleichzeitig an der Stufe 66 anliegt (Fig. 1a). Der Innendurchmesser des schlauchförmigen Abschnitts 13 und des Wulstes 14 ist in entspanntem Zustand des Materials kleiner als der Außendurchmesser des Käfigs 52, des Überwurfrings 60 und der Verjüngung 45. Zur Montage muss der schlauchförmige Abschnitt 13 und der Wulst 14 somit aufgedehnt werden. Nach dem Überwinden der Stufe 66 wird die Dehnung wieder reduziert. Somit ist der Ballon 12 schon vor dem Schließen des Klemmelementes 18 vorläufig gegen Verrutschen gesichert. Durch Drehen des Drehringes 23 wird das Klemmelement (18) in geschlossene Stellung bewegt, d. h. der Wulst 14 wird mit der gewünschten Kraft zwischen der Stufe 66 und der Kontaktfläche 111 des Ringes 110 eingeklemmt (Fig. 1).

In einem zweiten Ausführungsbeispiel ist der Ballon 11, wie in Fig. 5 im Längsschnitt dargestellt, mit einer Montagehilfe 85 versehen. Die Montagehilfe 85 besteht aus einem Rohr aus transparentem Kunststoff, dessen Innendurchmesser etwas größer als der Außendurchmesser des Käfigs 52 und des Überwurfrings 60 ist. Die Montagehilfe 85 ist kürzer als der schlauchförmige Abschnitt 13 des Ballons 11. Der Innendurchmesser des schlauchförmigen Endes 13 ist kleiner als der Außendurchmesser der Montagehilfe 85. Die Montagehilfe 85 dehnt somit den schlauchförmigen Abschnitt 13 auf. Der zur Öffnung hin überstehende Teil des schlauchförmigen Abschnitts 13 ist vor der Montage auf der Außenseite der Montagehilfe aufgerollt. Umfänglichen Erhebungen 87 und 88 auf der Oberfläche der Montagehilfe 85 verhindern ein ungewolltes Abrollen des aufgerollten Abschnitts des schlauchförmigen Abschnitts 13.

Der Ballon 11 des zweiten Ausführungsbeispiels ist ähnlich aufgebaut wie Ballon 12 des ersten Ausführungsbeispiels. Der tropfenförmige Ballonkörper wurde jedoch durch einen länglichen, zylinderförmigen ersetzt.

In einem dritten Ausführungsbeispiel ist der Aufbau des Ballontrokars distalseitig des Ventilkörpers modifiziert.

Den teilweise im Längsschnitt aufgebrochenen Abschnitt einer Seitenansicht einer Trokarhülse 15 gemäß dem dritten Ausführungsbeispiel zeigt Fig. 8. Die Haupthülse 89 ist wie die Haupthülse 43 des ersten und zweiten Ausführungsbeispiels mit dem Ventilkörper 68 verbunden. Das Außenrohr 90 umgibt die Haupthülse 89 vom Ventilkörper 68 bis zu dem Ring 94, der mit dem Außenrohr 90 verbunden ist. Ring 94 bildet die Stufe 95, gegen die der Wulst 14 des Ballons 10 zu dessen Befestigung gepresst werden kann.

Außenrohr 90 und Haupthülse 89 sind am proximalen Ende der Haupthülse 89 durch ein Außengewinde 89a auf der Außenseite der Haupthülse 89 und ein Innengewinde 90a auf der Innenseite des Außenrohres 90 miteinander verschraubt.

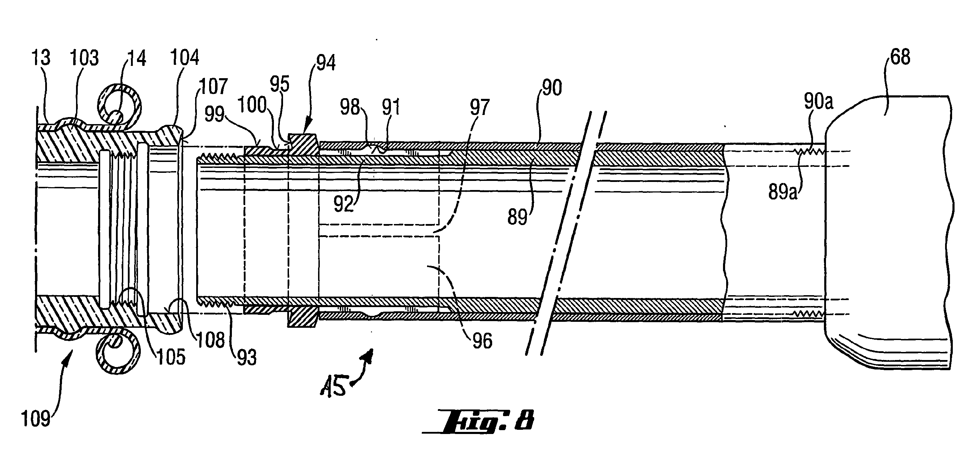

Das Klemmelement 102 ist, wie in Fig. 7 dargestellt, als distale Führung ausgebildet. Dieses wird über dessen Innengewinde 105 und das am distalen Ende der in Fig. 8 dargestellten Haupthülse 89 angeordnete Außengewinde 93 auf die Haupthülse 89 aufgeschraubt. Der Wulst 14 eines zu befestigenden Ballons 11 wird durch die Kontaktfläche 107 des Klemmelements 102 gegen die Stufe 95 gepresst. Die Kontaktfläche 107 ist gegen die Oberfläche der Trokarhülse 15 geneigt. Der Anpressdruck kann durch Drehen des Klemmelements 102 eingestellt werden.

Das Klemmelement 102 ist, wie in Fig. 7 dargestellt, einstückig aus transparentem Kunststoff gefertigt. Im distalen Bereich 106 ist der Außendurchmesser des Klemmelements 102 verringert. Durch das transparente Material wird die Beobachtung mit einem in die Trokarhülse eingeführten Endoskop ermöglicht. Die vormontierte Einheit aus Klemmelement 102 und Ballon 11 wird als Einmalprodukt bereitgestellt.

Der Innendurchmesser des schlauchförmigen Abschnitts 13 des Ballons 11 ist kleiner als der Außendurchmesser des proximalen Bereichs 109 des Klemmelements 102. Bei der Montage des Ballons 11 im proximalen Bereich 109 wird somit der schlauchförmige Abschnitt 13 aufgedehnt. Der zur Öffnung hin überstehende Teil des schlauchförmigen Abschnitts 13 ist, vor dem Aufschrauben des Klemmelements 102, auf deren Außenseite aufgerollt. Zwei ringförmige Erhebungen 103 und 104 auf der Oberfläche des proximalen Bereichs 109 verhindern ein ungewolltes Abrollen des aufgerollten Abschnitts des schlauchförmigen Abschnitts 13.

Die Stufe 95, gegen die der Wulst 14 des Ballons 11 durch das Klemmelement gepresst wird, ist Teil des Ringes 94. Stufe 95 ist gegen die Oberfläche der Trokarhülse 15 geneigt.

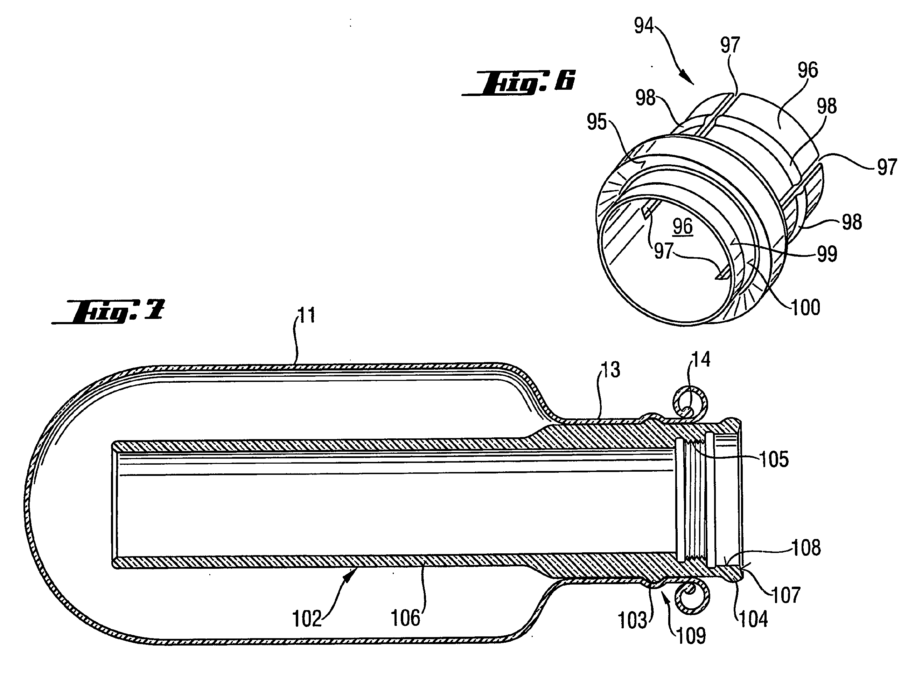

Eine perspektivische Darstellung des Ringes 94 zeigt Fig. 6. Wie bereits beim ersten Ausführungsbeispiel bei der Befestigung des Ringes 110 an dem Außenrohr 38 beschrieben, greifen vier Laschen des Rings 94, die in ihrer Gesamtheit mit der Bezugsziffer 96 bezeichnet werden, zwischen die Außenseite der Haupthülse 89 und die Innenseite des Außenrohres 90. Die Laschen 96 werden durch die Kerben 97, die in ihrer Gesamtheit mit der Bezugsziffer 96 bezeichnet werden, voneinander getrennt. Der für die Laschen 96 benötigte Zwischenraum wird durch die Verjüngung 92 des Außendurchmessers der Haupthülse 89 geschaffen.

Die umfänglich an den Außenseiten der Laschen ausgebildeten Erhebungen, die in ihrer Gesamtheit mit der Bezugsziffer 98 bezeichnet werden, greifen, wie beim ersten Ausführungsbeispiel beschrieben, zur Fixierung des Ringes in die Ringnut 91, die an der Innenseite des Außenrohres 90 ausgebildet ist. Der Ring 94 ist somit zur Längsachse der Trokarhülse drehbar in der Haupthülse 89 gelagert.

Der Ring 94 weist in distaler Richtung eine Auflage 99 auf, die sich an die Stufe 95 anschließt. Die Auflage 99 weist in direktem Anschluss an die Stufe 95, wie in Fig. 6 und 8 dargestellt, eine umfängliche Vertiefung 100 auf. Ring 94 wird, wie Ring 110, aus Kunststoff oder aus Stahl gefertigt.

Wird das Klemmelement 102 auf die Haupthülse 89 aufgesetzt, ragt die Auflage 99 in die Öffnung 108 des Klemmelements 102.

Beim Abrollen des aufgerollten Abschnitts des schlauchförmigen Abschnitts 13 wird somit sichergestellt, dass der Wulst 14 des Ballons 12 auf der Auflage 99 zu liegen kommt. Das Festklemmen des Wulstes 14 erfolgt durch Drehen des Klemmelements 102. Durch die Auflage 99 wird ermöglicht, dass während des Festklemmens der Wulst 14 der Drehbewegung des Klemmelements 102 folgen kann. So wird eine Beschädigung des Wulstes 12 durch Scherkräfte vermieden. Der Wulst 12 wird beim Festklemmen entlang der Auflage 99 geschoben, bis er in der Vertiefung 100 einrastet und bis der gewünschte Anpressdruck erreicht ist.

## Patentansprüche

1. Ballontrokar (10) bestehend aus einer Trokarhülse (15, 16) und einem Ballon (11, 12), der ein distales Ende der Trokarhülse (15, 16) umgeben kann, **dadurch gekennzeichnet, dass** der Ballon (11, 12) zur Fixierung an der Trokarhülse (15, 16) durch ein Klemmelement (18, 102) gegen eine an der Trokarhülse (15, 16) umfänglich ausgebildete Stufe (66, 95) gepresst werden kann, wobei das Klemmelement (18, 102) durch Drehung zumindest eines Teils des Klemmelements (18, 102) um eine Längsachse der Trokarhülse (15, 16) in Längsrichtung zur Trokarhülse (15, 16) bewegbar ist.

2. Ballontrokar (10) nach Anspruch 1, wobei das Klemmelement (18, 102) auf jener Seite der Stufe (66, 95) an der Trokarhülse (15, 16) fixierbar ist, von welcher der Ballon (11, 12) gegen die Stufe (66, 95) gepresst werden kann.

3. Ballontrokar (10) nach einem der Ansprüche 1 bis 2, wobei das Klemmelement (18, 102) zumindest abschnittsweise ring- oder röhrenförmig ausgebildet ist, wobei dessen Querschnitt zumindest abschnittsweise eine Öffnung aufweist, deren Form dem äußeren Querschnitt der Trokarhülse (15, 16) entspricht.

4. Ballontrokar (10) nach einem der Ansprüche 1 bis 3, wobei das Klemmelement (18, 102) umfänglich eine Kontaktfläche (107, 111) aufweist, die zur Fixierung des Ballons (11, 12) an dessen Oberfläche anliegt.

5. Ballontrokar (10) nach Anspruch 4, wobei die Kontaktfläche (107, 111) als Stirnfläche des Klemmelements (18, 102) ausgebildet ist.

6. Ballontrokar (10) nach einem der Ansprüche 4 bis 5, wobei die Kontaktfläche (107, 111) zumindest abschnittsweise gegen die Oberfläche der Trokarhülse (15, 16) geneigt ist.

7. Ballontrokar nach einem der Ansprüche 1 bis 6, wobei die Stufe (95) zumindest abschnittsweise gegen die Oberfläche der Trokarhülse (15) geneigt ist.

8. Ballontrokar nach einem der Ansprüche 1 bis 7, wobei die Stufe (95) drehbar zur Längsachse der Trokarhülse (15) gelagert ist.

9. Ballontrokar (10) nach einem der Ansprüche 1 bis 6, wobei das Klemmelement (18) eine Handhabe (20) zu dessen Betätigung aufweist.

10. Ballontrokar (10) nach Anspruch 9, wobei die Handhabe (20) als Drehring (23) ausgebildet ist, durch dessen Drehung das Klemmelement (18) in axialer Richtung bewegt wird.

11. Ballontrokar (10) nach einem der Ansprüche 9 bis 10, wobei die Handhabe (20) am proximalen Ende der Trokarhülse (16) angeordnet ist.

12. Ballontrokar nach einem der Ansprüche 1 bis 8, wobei das Klemmelement (102) zumindest abschnittsweise innerhalb des Ballons (11) angeordnet ist.

13. Ballontrokar nach einem der Ansprüche 1 bis 8 oder 12, wobei das Klemmelement (102) zur Fixierung des Ballons (11) in Richtung des proximalen Endes der Trokarhülse (15) bewegt wird.

14. Ballontrokar nach einem der Ansprüche 1 bis 8 oder 12 bis 13, wobei das Klemmelement (102) als eine distale Führung der Trokarhülse (15) ausgebildet ist.

15. Ballontrokar (10) nach einem der Ansprüche 1 bis 6 oder 9 bis 11, wobei eine Öffnung des Ballons (11) durch eine Montagehilfe (85) aufgedehnt wird, wobei der Innendurchmesser der Montagehilfe (85) größer oder gleich dem Außendurchmesser der Trokarhülse (16) ist.

16. Ballontrokar (10) nach einem der Ansprüche 1 bis 15, wobei ein Rand einer Öffnung des Ballons (11, 12) als Wulst (14) ausgebildet ist.

## Claims

1. Balloon trocar (10) comprising a trocar sleeve (15, 16) and a balloon (11, 12) which can surround a distal end of the trocar sleeve (15, 16), **characterized in that** the balloon (11, 12) is fixed to the trocar sleeve (15, 16) by being pressed by a clamping element (18, 102) against a circumferential step (66, 95) formed on the trocar sleeve (15, 16), the clamping element (18, 102) being able to be moved in the longitudinal direction to the trocar sleeve (15, 16) by means of at least part of the clamping element (18, 102) being rotated about a longitudinal axis of the trocar sleeve (15, 16).

2. Balloon trocar (10) according to Claim 1, the clamping element (18, 102) being able to be fixed to the trocar sleeve (15, 16) on that side of the step (66, 95) from which the balloon (11, 12) can be pressed against the step (66, 95).

3. Balloon trocar (10) according to either of Claims 1 and 2, at least part of the clamping element (18, 102) being annular or tubular in configuration, and at least part of its cross section having an opening whose shape corresponds to the outer cross section of the trocar sleeve (15, 16).

4. Balloon trocar (10) according to one of Claims 1 to 3, the clamping element (18, 102) having a circumferential contact surface (107, 111) which fixes the balloon (11, 12) by bearing on the surface of the latter.

5. Balloon trocar (10) according to Claim 4, the contact surface (107, 111) being configured as a front face of the clamping element (18, 102).

6. Balloon trocar (10) according to one of Claims 4 to 5, at least part of the contact surface (107, 111) being inclined relative to the surface of the trocar sleeve (15, 16).

7. Balloon trocar according to one of Claims 1 to 6, at least part of the step (95) being inclined relative to the surface of the trocar sleeve (15).

8. Balloon trocar according to one of Claims 1 to 7, the step (95) being mounted so that it can rotate relative to the longitudinal axis of the trocar sleeve (15).

9. Balloon trocar (10) according to one of Claims 1 to 6, the clamping element (18) having a handle (20) for operating it.

10. Balloon trocar (10) according to Claim 9, the handle (20) being configured as a rotary ring (23) whose rotation moves the clamping element (18) in the axial direction.

11. Balloon trocar (10) according to one of Claims 9 to 10, the handle (20) being arranged on the proximal end of the trocar sleeve (16).

12. Balloon trocar according to one of Claims 1 to 8, at least part of the clamping element (102) being arranged inside the balloon (11).

13. Balloon trocar according to one of Claims 1 to 8 or 12, the clamping element (102) being moved in the direction of the proximal end of the trocar sleeve (15) in order to fix the balloon (11).

14. Balloon trocar according to one of Claims 1 to 8 or 12 to 13, the clamping element (102) being configured as a distal guide of the trocar sleeve (15) .

15. Balloon trocar (10) according to one of Claims 1 to 6 or 9 to 11, an opening of the balloon (11) being expanded by an assembly aid (85), the internal diameter of the assembly aid (85) being greater than or equal to the external diameter of the trocar sleeve (16).

16. Balloon trocar (10) according to one of Claims 1 to 15, en edge of an opening of the balloon (11, 12) being configured as a bead (14).

## Revendications

1. Trocart à ballon (10), composé d'un manchon de trocart (15, 16) et d'un ballon (11, 12) qui peut entourer une extrémité distale du manchon de trocart (15, 16),
**caractérisé en ce que**
pour la fixation au manchon de trocart (15, 16), le ballon (11, 12) peut être comprimé par un élément de serrage (18, 102) contre un palier (66, 95) réalisé de façon circonférentielle sur le manchon de trocart (15, 16), l'élément de serrage (18, 102) étant mobile par rotation au moins d'une partie de l'élément de serrage (18, 102) autour d'un axe longitudinal du manchon de trocart (15, 16) dans la direction longitudinale par rapport au manchon de trocart (15, 16).

2. Trocart à ballon (10) selon la revendication 1, dans lequel l'élément de serrage (18, 102) peut être fixé au manchon de trocart (15, 16) du côté du palier (66, 95) à partir duquel le ballon (11, 12) peut être comprimé contre le palier (66, 95).

3. Trocart à ballon (10) selon la revendication 1 ou 2, dans lequel l'élément de serrage (18, 102) est réalisé au moins par endroits en forme d'anneau ou de tube, dans lequel la section transversale de celui-ci présente au moins par endroits une ouverture dont la forme correspond à la section transversale extérieure du manchon de trocart (15, 16).

4. Trocart à ballon (10) selon l'une quelconque des revendications 1 à 3, dans lequel l'élément de serrage (18, 102) présente de façon circonférentielle une surface de contact (107, 111) qui est adjacente à la surface du ballon (11, 12) pour la fixation de celui-ci.

5. Trocart à ballon (10) selon la revendication 4, dans lequel la surface de contact (107, 111) est réalisée comme une surface frontale de l'élément de serrage (18, 102).

6. Trocart à ballon (10) selon l'une quelconque des revendications 4 à 5, dans lequel la surface de contact (107, 111) est au moins par endroits inclinée par rapport à la surface du manchon de trocart (15, 16).

7. Trocart à ballon (10) selon l'une quelconque des revendications 1 à 6, dans lequel le palier (95) est au moins par endroits incliné par rapport à la surface du manchon de trocart (15).

8. Trocart à ballon (10) selon l'une quelconque des revendications 1 à 7, dans lequel le palier (95) est logé de façon pivotante par rapport à l'axe longitudinal du manchon de trocart (15).

9. Trocart à ballon (10) selon l'une quelconque des revendications 1 à 6, dans lequel l'élément de serrage (18) présente un dispositif de manipulation (20) pour l'actionnement de celui-ci.

10. Trocart à ballon (10) selon la revendication 9, dans lequel le dispositif de manipulation (20) est réalisé comme une bague tournante (23) dont la rotation permet de déplacer l'élément de serrage (18) dans la direction axiale.

11. Trocart à ballon (10) selon la revendication 9 ou 10, dans lequel le dispositif de manipulation (20) est disposé à l'extrémité proximale du manchon de trocart (16).

12. Trocart à ballon (10) selon l'une quelconque des revendications 1 à 8, dans lequel l'élément de serrage (102) est au moins par endroits disposé à l'intérieur du ballon (11).

13. Trocart à ballon (10) selon l'une quelconque des revendications 1 à 8 ou 12, dans lequel, pour la fixation du ballon (11), l'élément de serrage (102) est déplacé en direction de l'extrémité proximale du manchon de trocart (15).

14. Trocart à ballon (10) selon l'une quelconque des revendications 1 à 8 ou 12 à 13, dans lequel l'élément de serrage (102) est réalisé comme un guidage distal du manchon de trocart (15).

15. Trocart à ballon (10) selon l'une quelconque des revendications 1 à 6 ou 9 à 11, dans lequel une ouverture du ballon (11) est évasée par un accessoire de montage (85), dans lequel le diamètre intérieur de l'accessoire de montage (85) est supérieur ou égal au diamètre extérieur du manchon de trocart (16).

16. Trocart à ballon (10) selon l'une quelconque des revendications 1 à 15, dans lequel un bord d'une ouverture du ballon (11, 12) est réalisé comme un bourrelet (14).
